Europäisches Patentamt

**European Patent Office**

Office européen des brevets

(19)

(11) Veröffentlichungsnummer : **0 043 530**
**B1**

(12)
# EUROPÄISCHE PATENTSCHRIFT

(45) Veröffentlichungstag der Patentschrift :
**16.11.83**

(51) Int. Cl.³ : **C 12 Q   1/62**

(21) Anmeldenummer : **81105022.8**

(22) Anmeldetag : **29.06.81**

(54) **Verfahren und Reagenz zur Bestimmung von Harnsäure.**

(30) Priorität : **03.07.80 DE 3025170**

(43) Veröffentlichungstag der Anmeldung :
**13.01.82 Patentblatt 82/02**

(45) Bekanntmachung des Hinweises auf die Patenterteilung : **16.11.83 Patentblatt 83/46**

(84) Benannte Vertragsstaaten :
**AT BE CH DE FR IT LI LU NL SE**

(56) Entgegenhaltungen :
**DE A 2 450 726**
**DE A 2 718 588**
**US A 3 746 625**
**US A 3 956 069**
**US A 4 189 536**
**US A 4 229 369**

**CLINICAL CHEMISTRY, Band 25,-April 1979, Nr. 4, EASTON, PENNSYLVANIA (US) KNUT BARTL et al. : « Improved Automated Kinetic Determination of Uric Acid in Serum by Use of Uricase/Catalese/Aldehyde Dehydrogenase » Seiten 619-621**

(73) Patentinhaber : **C.H. BOEHRINGER SOHN**
**Postfach 200**
**D-6507 Ingelheim am Rhein (DE)**

(72) Erfinder : **Gorka, Günther, Dr.**
**Am Rebenhang 28**
**D-6200 Wiesbaden-Auringen (DE)**
Erfinder : **Stinshoff, Klaus, Dr.**
**5, Chemin de Commanderie**
**CH-1228 Plane-ies-Ouates (CH)**

**0 043 530**

Verfahren and Reagenz zur Bestimmung von Harnsäure

Die quantitative Analyse der Harnsäure in biologischem Material wie Serum oder Plasma gehört zu den häufig durchzuführenden Untersuchungen und spielt daher eine wichtige Rolle in der klinischen Chemie.

Die enzymatische Bestimmung der Harnsäure erfolgte bisher vorwiegend nach der Uricase-Methode, die als Referenzmethode gilt, und nach dem Verfahren von Kageyama (Clin. Chim. Acta 31, 421 (1971), vergleiche auch Bergmeyer, « Methoden der enzymatischen Analyse », Band 2, (1974), 1999-2005, Verlag Chemie).

Beide Verfahren sind jedoch mit Nachteilen behaftet. Bei der Uricase-Methode steht einer hohen Eigenextinktion der Serumprobe bei 293 nm ein relativ kleines Signal aufgrund der Harnsäureabnahme gegenüber ; außerdem wird bei einer Wellenlänge gearbeitet, für die im Routinelabor keine Ausrüstung bereitsteht. Beim Verfahren von Kageyama wird das durch Einwirkung der Uricase gebildete $H_2O_2$ zur Oxydation von Methanol zu Formaldehyd verwandt ; der Formaldehyd reagiert mit Acetylaceton und Ammoniak unter Bildung von 3,5-Diacetyl-1,4-dihydrolutidin. Zum Abschluß der Reaktion werden jedoch 60 Minuten Zeit bei erhöhter Temperatur (37 °C) benötigt, was für Analysenautomaten einen entscheidenden Nachteil darstellt.

Neuerdings wurde ein Verfahren bekannt (DE-A-24 50 726), bei dem das entstehende $H_2O_2$ über Katalase und Aldehyddehydrogenase bestimmt wird, wobei aus NAD(P) NAD(P)H entsteht, das photometrisch bei 334, 340 oder 366 nm gemessen werden kann.

Besonders zur Bestimmung von Harnsäure erweist sich dieses Verfahren gegenüber bisher verwendeten als vorteilhaft, da nur noch 15 Minuten Zeit gegenüber früher 60 Minuten (Bergmeyer, H.U., loc. cit., S. 2002-2005), benötigt wird ; hierdurch ist diese Methode besonders für Analysenautomaten geeignet.

Bei der Anwendung dieses Verfahrens stellte sich nun heraus, daß im Serum Bestandteile enthalten sind, die die Aldehyddehydrogenase desaktivieren. So konnte gefunden werden, daß nach einer Inkubationsdauer von Serum mit Reagenz ohne Uricase von 4 Minuten und anschließender Zugabe von Uricase die Reaktion nach ca. 10 Minuten beendet ist. Wenn 30 Minuten vorinkubiert wird, kommt nach Zugabe von Uricase die Reaktion oft erst nach 20-30 Minuten zu einem Endpunkt, oder sie geht in eine sich längere Zeit fortsetzende Schleichreaktion über. Erschwerend kommt hinzu, daß die Abnahme der Aldehyddehydrogenase-Aktivität bei verschiedenen Seren mit unterschiedlicher Geschwindigkeit abläuft.

Vor allem für den Einsatz des Reagenz an Analysenautomaten ist dies ein großer Nachteil. Hier sind oft gerätebedingt längere Vorinkubationszeiten notwendig, bevor Uricase als Startreagenz zugegeben wird. Die Abnahme der Aldehyddehydrogenase-Aktivität kann dann dazu führen, daß die Reaktion in der vorgegebenen Zeit nicht abläuft.

Bei Analysenautomaten, die Substrate auf kinetischer Basis bestimmen, stört der aufgezeigte Sachverhalt in noch stärkerem Maße. Da hier die Geschwindigkeit der Reaktion gemessen wird, muß die Aktivität der beteiligten Enzyme während der Meßperiode konstant bleiben ; falls dies nicht der Fall ist, wird die Reaktionsgeschwindigkeit nicht nur von der Menge des vorhandenen Substrats, sondern auch von der wechselnden Aktivität der beteiligten Enzyme beeinflußt und damit verfälscht. Insbesondere dann, wenn die Aldehyddehydrogenase-Reaktion den geschwindigkeitsbestimmenden Schritt der Gesamtreaktion darstellt, ist eine gleichbleidende Aldehyddehydrogenase-Aktivität sicherzustellen.

Aus der Literatur (Black, S., Arch. Biochem. Biophys., 34 (1951), 86-97 ; Steinmann, C.R., und Jacoby, W.B., J. Biol. Chem., 242 (1967), 5019-5023) ist bekannt, daß es sich bei der Aldehyddehydrogenase um ein Thiolgruppenhaltiges Enzym handelt, das mit Thiol-Reagenzien und/oder mit Komplexbildnern stabilisierbar ist. Aufgeführt seien hier EDTA, Cystein, Glutathion, Mercapto-Äthanol, Thioglycolsäure. EDTA ist als Schutzreagenz in Gegenwart von Serum nicht ausreichend wirksam. Die anderen angeführten Reagenzien schützen zwar die Aldehyddehydrogenase, führen aber in der Inkubationsmischung zu einer Schleichreaktion, die je nach Konzentration des zugesetzten Stoffes und je nach untersuchtem Serum zwischen 0,001 und 0,010 Extinktionen/Minute liegt :

| Zusatz | Konzentration im Test (Mol/l) | Schleichreaktion ($\Delta E/min$) |
|---|---|---|
| Dithiothreitol | $5 \times 10^{-4}$ | 0,002-0,003 |
| Cystein | $5 \times 10^{-4}$ | 0,005-0,007 |
| Glutathion | $5 \times 10^{-4}$ | 0,004-0,007 |
| Thioglycolsäure | $5 \times 10^{-4}$ | 0,001-0,002 |

Überraschenderweise wurde nun gefunden, daß bei Verfahren zur Bestimmung von Harnsäure in biologischem Material mittels Uricase Katalase und Aldehyddehydrogenase in gegenwart von « Athanol und Nicotinamid-adenin-dinucleotid bzw. -phosphat (NAD(P)) durch Zusatz von 2-Mercaptobernsteinsäure die Aldehyddehydrogenase geschützt wird und im Gegensatz zu anderen Thiol-Reagenzien keine Schleichreaktion eintritt.

Durch Zusatz dieses Reagenzes ist es jetzt möglich, Aldehyddehydrogenase-abhängige Reaktionen störungsfrei an Analysenautomaten und manuell durchzuführen. Außerdem ist bei längeren Vorinkubationszeiten — im Gegensatz zu den bekannten Verfahren — die Zeit bis zur Erreichung des Endpunktes nicht verlängert.

Die in der DE-B-27 18 588 zur Unterdrückung von Schleichreaktionen genannten Stoffe beziehen sich vorwiegend auf urämische Proben, in denen bestimmte Enzyme in erhöhter Aktivität vorkommen können. Deren Einfluß wird durch Zusatz der in der DE-B-27 18 588 genannten Stoffe unterdrückt (Clin. Chem. 25/4, 619-621 (1979)). Diese Stoffe bewirken keinen Schutz der Aldehyddehydrogenase.

Die erfindungsgemäß verwendete 2-Mercaptobernsteinsäure dagegen schützt die für den Nachweis der Harnsäurereaktion entscheidende Aldehyddehydrogenase vor desaktivierenden Substanzen, die in jedem Serum vorkommen, ohne daß dafür eine unterschiedlich ausgeprägte Schleichreaktion in Kauf genommen werden muß. Sie unterscheidet sich daher in ihrem Wirkort und in ihrer Wirkung wesentlich von den in der DE-B-27 18 588 genannten Substanzen.

Ein bevorzugtes Reagenz hat folgende Zusammensetzung :

25 bis 500 U/l Uricase, 300 bis 1000 k U/l Katalase, 100 bis 500 U/l Aldehyddehydrogenase, 0,5 bis 2 Mol/l Äthanol, 0,2 bis 1,5 mMol/l $NAD^+$ oder $NADP^+$, 0,01 bis 0,5 Mol/l Oxamsäure, 20 bis 100 mMol/l Puffer pH 6,5 bis 9 und 0,000 1 bis 0,1 Mol/l 2-Mercaptobernsteinsäure.

Der pH-Wert liegt vorzugsweise zwischen 8 und 8,5.

Das folgende Beispiel dient zu näheren Erläuterung der Erfindung :

Beispiel

Reagenz 1 : Kaliumdihydrogenphosphat-Puffer
(42 mMol/l, pH 8,0),
Äthanol (1,43 mMol/l),
$NADP^+$ (0,265 mMol/l),
Katalase (350 k U/l),
Aldehyddehydrogenase (250 U/l),
2-Mercaptobernsteinsäure (wechselnde Konzentrationen).

Reagenz 2 : Uricase (5 k U/l)

Bestimmungsansatz für Harnsäure

Meßstrahlung 334 nm, 340 nm, 365 nm ; Schichtdicke 1 cm, Temperatur 25 °C.

1 ml Reagenz 1 in Küvette pipettieren, 50 µl Probe zusetzen und mischen. Extinktion am Schreiber registrieren, $E_1$ nach einer definierten Vorinkubationszeit ablesen (ca. 4 Minuten), dann 10 µl Reagenz 2 zugeben, nach 15 Minuten $E_2$ bestimmen.

Berechnung der Konzentration (C) der Harnsäure :

$C = (E_2$ minus $E_1) \times 3,43$ mMol/l (Hg 334 nm)
$C = (E_2$ minus $E_1) \times 3,36$ mMol/l (Hg 340 nm)
$C = (E_2$ minus $E_1) \times 6,06$ mMol/l (Hg 365 nm, NADP)

Versuch 1

Mit verschiedenen Seren wurden unter Verwendung von Reagenz 1 verschiedene Vorinkubationszeiten vor Zugabe von Reagenz 2 eingehalten. Hierbei zeigte sich, daß die Zeit bis zum Erreichen des Endpunkts bei längeren Vorinkubationszeiten (bis 30 Minuten) nicht signifikant größer war als bei 4 Minuten Vorinkubationszeit. Die beiliegenden Abbildungen demonstrieren den Unterschied im Testverlauf ohne und mit 2-Mercaptobernsteinsäure.

Versuch 2

Mit einem Reagenz obiger Zusammensetzung wurde eine Haltbarkeitsprüfung bei Raumtemperatur und Kühlschranktemperatur durchgeführt. Als Kriterium wurde die Zeit bis zum Erreichen des Endpunkts in der Harnsäurebestimmung gewählt. Wenn diese Zeit 15 Minuten überstieg, wurde das Reagenz als nicht länger verwendbar definiert.

Die Haltbarkeit beträgt beim Zusatz von ca. 1 bis 5 mMol 2-Mercaptobernsteinsäure bei Raumtemperatur mindestens 7 Tage, bei Kühlschranktemperatur mindestens 14 Tage. Diese Haltbarkeit liegt deutlich über der bisher angegebenen von einem Tag bei Raumtemperatur und 8 Tagen Kühlschranktemperatur.

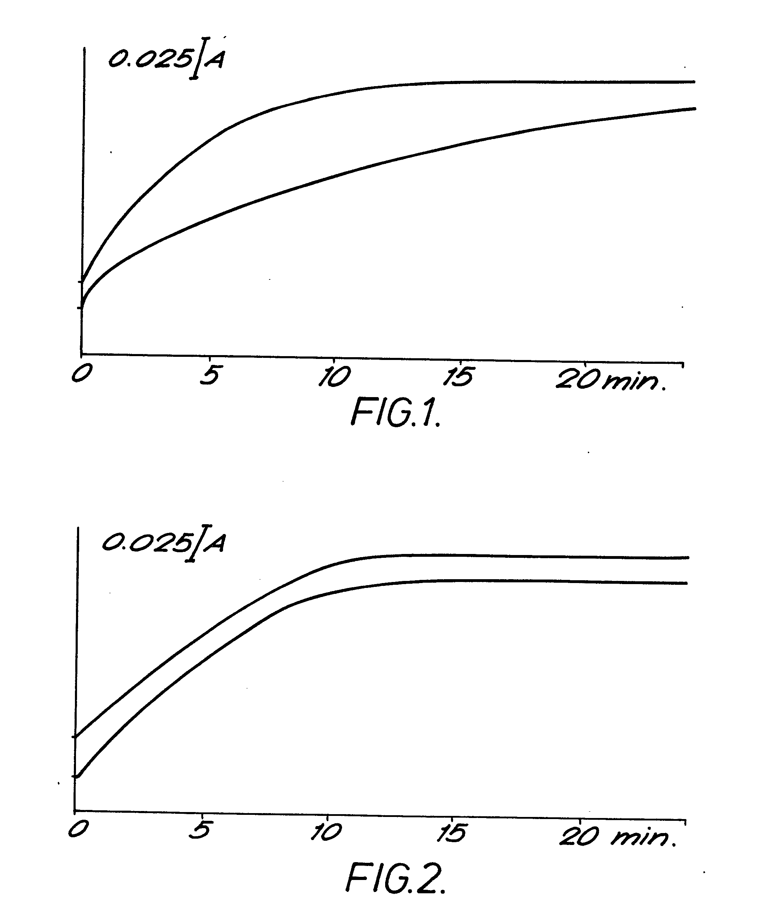

**Ansprüche**

1. Verfahren zur Bestimmung von Harnsäure in biologischem Material mittels Uricase, Katalase und

3

Aldehyddehydrogenase in Gegenwart von Äthanol und Nicotinamid-adenin-dinucleotid beziehungsweise -phosphat (NAD(P)), dadurch gekennzeichnet, daß dem Testansatz 2-Mercaptobernsteinsäure zugesetzt wird.

2. Verfahren nach Anspruch 1, dadurch gekennzeichnet, daß 0,000 1 bis 0,1 Mol/l 2-Mercaptobernsteinsäure verwendet werden.

3. Verfahren nach den Ansprüchen 1 und 2, dadurch gekennzeichnet, daß man bei pH-Werten zwischen 6,5 und 9, vorzugsweise zwischen 8 und 8,5, arbeitet.

4. Reagenz zur Bestimmung von Harnsäure, enthaltend Uricase, Katalase, Aldehyddehydrogenase, Äthanol, NAD(P) und Puffer, dadurch gekennzeichnet, daß das Reagenz die Verbindung 2-Mercaptobernsteinsäure enthält.

5. Reagenz nach Anspruch 4, dadurch gekennzeichnet, daß es zusätzlich Oxamsäure enthält.

6. Reagenz nach den Ansprüchen 4 und 5, dadurch gekennzeichnet, daß es etwa 25 bis 500 U/l Uricase, 300 bis 1 000 k U/l Katalase, 100 bis 500 U/l Aldehyddehydrogenase, 0,5 bis 2 Mol/l Äthanol, 0,2 bis 1,5 mMol/l NAD$^+$ oder NADP$^+$, 0,1 bis 0,5 Mol/l Oxamsäure, 20 bis 100 mMol/l Puffer pH 6,5 bis 9 und 0,000 1 bis 0,1 Mol/l 2-Mercaptobernsteinsäure enthält.


## Claims

1. Process for detecting uric acid in biological material using uricase, catalase and aldehyde dehydrogenase in the presence of ethanol and nicotinamide adenine dinucleotide or nicotinamide adenine dinucleotide phosphate (NAD(P)), characterised in that 2-mercapto-succinic acid is added to the test mixture.

2. Process as claimed in claim 1, characterised in that 0.000 1 to 0.1 mole/litre of 2-mercaptosuccinic acid is used.

3. Process as claimed in claim 1, characterised in that the work is done at pH values of between 6.5 and 9, preferably between 8 and 8.5.

4. Reagent for detecting uric acid, containing uricase, catalase, aldehyde dehydrogenase, ethanol, NAD(P) and buffer, characterised in that the reagent contains the compound 2-mercaptosuccinic acid.

5. Reagent as claimed in claim 4, characterised in that it additionally contains oxamic acid.

6. Reagent as claimed in claims 4 and 5, characterised in that it contains about 25 to 500 U/l of uricase, 300 to 1 000 kU/l of catalase, 100 to 500 U/l of aldehyde dehydrogenase, 0.5 to 2 mole/l of ethanol, 0.2 to 1.5 mmole/l of NAD$^+$ or NADP$^+$, 0.1 to 0.5 mole of oxamic acid, 20 to 100 mmole/l of buffer (pH 6.5 to 9) and 0.000 1 to 0.1 mole/l of 2-mercaptosuccinic acid.


## Revendications

1. Procédé pour la détermination de l'acide urique dans la matière biologique au moyen d'uricase, de catalase et d'aldéhydedeshydrogénase en présence d'éthanol et de nicotinamide-adénine-dinucléotide ou -phosphate (NAD(P)), caractérisé en ce que de l'acide 2-mercaptosuccinique est ajouté au mélange d'essai.

2. Procédé selon la revendication 1, caractérisé en ce qu'on utilise 0,000 1 à 0,1 mole/l d'acide 2-mercaptosuccinique.

3. Procédé selon les revendications 1 et 2, caractérisé en ce qu'on travaille à des valeurs de pH entre 6,5 et 9, de préférence entre 8 et 8,5.

4. Réactif pour la détermination de l'acide urique, contenant de l'uricase, de la catalase, de l'aldéhydedeshydrogénase, de l'éthanol, du NAD(P) et du tampon, caractérisé en ce que le réactif contient le composé acide 2-mercaptosuccinique.

5. Réactif selon la revendication 4, caractérisé en ce qu'il contient en outre de l'acide oxamique.

6. Réactif selon les revendications 4 et 5, caractérisé en ce qu'il contient environ 25 à 500 U/l d'uricase, 300 à 1 000 kU/l de catalase, 100 à 500 U/l d'aldéhydedeshydrogénase, 0,5 à 2 mole/l d'éthanol, 0,2 à 1,5 mmole/l de NAD$^+$ ou de NADP$^+$, 0,1 à 0,5 mole/l d'acide oxamique, 20 à 100 mmole/l de tampon pH 6,5 à 9 et 0,000 1 à 0,1 mole/l d'acide 2-mercaptosuccinique.

FIG.1.

FIG.2.